# EUROPEAN PATENT SPECIFICATION

(11) **EP 1 955 730 B1**
(45) Date of publication and mention of the grant of the patent: **23.12.2009**
(21) Application number: 08000073.0
(22) Date of filing: 03.01.2008
(51) Int. Cl.: A61N 1/26, A46B 15/00

(54) **Oral care device**
Mundpflegegerät
Dispositif de protection orale

(30) Priority: 31.01.2007 JP 2007021976
(43) Date of publication of application: 13.08.2008
(73) Proprietor: Panasonic Electric Works Co., Ltd., Kadoma-shi Osaka (JP)
(72) Inventor: Kitagawa, Tadanobu, Kadoma-shi Osaka 571-8686 (JP); Kitamura, Yoshihiro, Kadoma-shi Osaka 571-8686 (JP); Hoshino, Junichi, Kadoma-shi Osaka 571-8686 (JP)
(74) Representative: Appelt, Christian W.

(56) References cited:
- EP-A- 1 025 776
- WO-A-90/09206
- WO-A-2006/087927
- DE-A1- 3 637 183
- JP-A- 9 140 453
- US-A- 4 665 921
- US-A- 4 691 718
- US-A- 4 726 806
- US-A- 5 133 102

## Description

### TECHNICAL FIELD

The present invention relates to an oral care device supplying an electric current to an oral cavity for removal of tooth plaque.

### BACKGROUND ART

Japanese patent publication No. 9-140453 discloses an electric toothbrush configured to flow an electric current to teeth for removal of tooth plaque. The toothbrush has a handle and a brush head extending from the handle. The handle is provided with a touch electrode for contact with a user's hand, while the brush head is provided with a conductor held in an electrical contact with a brush. A battery is included in the handle to give a potential difference between the touch electrode and the conductor so as to flow an electric current to the oral cavity through a user's body for removing the tooth plaque. The touch electrode is made of a metal and is exposed on the exterior of the handle for electrical contact with the user's hand. During a prolonged use, however, the metal-made touch electrode suffers from elution of metal ions or corrosion, which might be somewhat harmful or deteriorate the appearance of the toothbrush, or even fail to flow the current consistently.

Document WO 90/09206 discloses a toothbrush for applying electrical stimulus to the gingival region having an electrode on the handle and an electrode having nibs at the base of the bristles. This document discloses the preamble of claim 1.

Furthermore, from document DE 36 37 183 A1 a neutral electrode for medical devices, especially dental surgical devices is known. The electrode is made of polyamide filled with carbon filaments.

### DISCLOSURE OF THE INVENTION

In view of the above problem, the present invention has been accomplished to provide an oral care device of which touch electrode is prevented from elution or corrosion for prolonged use without deterioration of the appearance and performance.

This object is solved by an oral care device according to claim 1. Claims 2 to 6 relate to a specifically advantageous realization of the oral care device according to claim 1.

The oral care device in accordance with the present invention includes a handle provided with a touch electrode for contact with a user's hand, a brush head extending from the handle and carrying a brush configured to be placed into an oral cavity of the user. The brush head is provided with a negative brush electrode disposed adjacent the brush and being configured to come into electrical contact with portions or substances in the oral cavity. A power source is accommodated within the handle to apply a voltage difference between the touch electrode and the brush electrode in order to flow a current within the oral cavity. The touch electrode is made of a non-metal material filled with carbon filaments. Accordingly, the touch electrode is free from any metal elution or corrosion while it is in contact with the user's hand to flow the current, which assure to keep the oral care device in good appearance and performance over a prolonged use.

Most preferably, the touch electrode is charged positive with respect to the brush electrode to charge the user's teeth positive, thereby attracting negative ions to the surface of the teeth for effectively decomposing and removing plaque therefrom. In this instance, the brush electrode is charged negative and is well prevented from elution in the oral cavity even when it is made of a metallic material.

The handle is configured to have a water-tight interior space within which the power source is disposed together with terminals respectively for electrical connection with said brush electrode and said touch electrodes The touch electrode is configured to have a portion which extends continuously into the interior space for electrical contact with a corresponding one of the terminals. Thus, the touch electrode can be held in direct electrical contact with the corresponding terminal, while the electrical connection can be concealed within the handle to be water-proofed.

In this connection, the touch electrode is molded as an integral part of the handle so that the handle can be easily shaped into a water-tight structure. Also with the integral molding, the handle can be free from any noticeable gap around the touch electrode, eliminating a possibility that any ionized material is trapped in the gap and therefore keeping the handle fee from contamination of the ionized material and associated lowering of the current flow from the touch electrode.

The touch electrode is composed of a non-electrically conductive matrix within which said carbon filaments are dispersed. The touch electrode may be electrically connected to the corresponding terminal by means of an electrically conductive adhesive. In this instance, the adhesive can permeate over the surface the conductive matrix for successful contact with the carboy fibers, thereby assuring a good electrical interconnection. Preferably, the adhesive is prepared in the form of a tap for easy connection of the touch electrode to the terminal. A screw may be used in combination with the tape to fasten the touch electrode to the terminal. In this case, the screw head is disposed in a recess formed in the outer surface of the touch electrode, and is concealed therein by a cap fitted in the recess.

Alternatively, the touch electrode may be electrically connected to the corresponding terminal by means of the screw without the use of the adhesive. In this instance, the tightening of the screw develops a force of squeezing the carbon fibers for electrical contact with the terminal, thereby assuring reliable electrical connection between the touch electrode and the terminal. Also in this case, the touch electrode may be formed with a recess for concealing therein the screw head.

These and still other advantageous features of the present invention will become apparent from the following detailed description of the embodiments when taken in conjunction with the attached drawings.

### BRIEF DESCRIPTION OF THE DRAWINGS

- FIG. 1: is a vertical section of an oral care device in accordance with a first embodiment of the present invention;
- FIG. 2: is a vertical section of an oral care device in accordance with a second embodiment of the present invention;
- FIG. 3: is a vertical section of an oral care device in accordance with a third embodiment of the present invention;
- FIG. 4: is a partial section of an oral care device in accordance with a fourth embodiment of the present invention;
- FIGS. 5A and 5C: are enlarged sectional views respectively illustrating aspects of the above embodiments;
- FIG. 6: is a partial section of an oral care device in accordance with a fifth embodiment of the present invention;
- FIG. 7: is an enlarged sectional view illustrating an electrical connection between a touch elec- trode and a corresponding terminal of the above embodiment;
- FIG. 8: is partial section of an oral care device in accordance with a sixth embodiment of the present invention;
- FIG. 9: is an enlarged sectional view illustrating an electrical connection between a touch elec- trode and a corresponding terminal of the above embodiment.

### BEST MODE FOR CARRYING OUT THE INVENTION

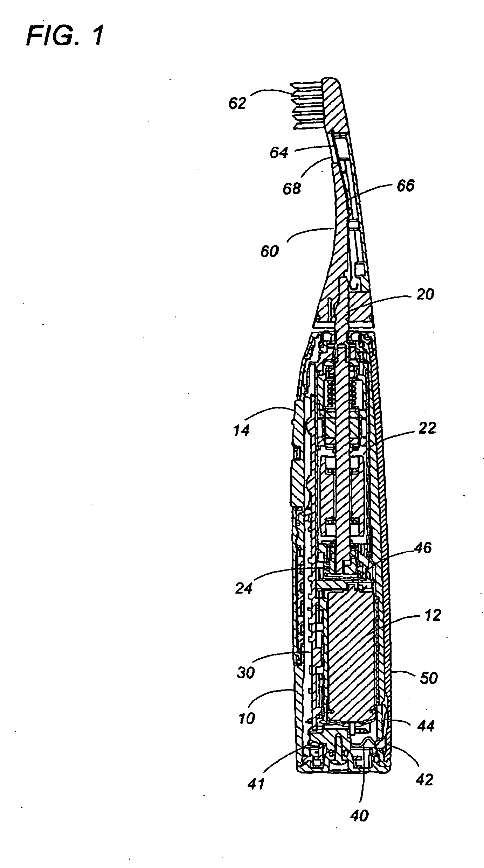

Referring now to FIG. 1, there is shown an oral care device which is provided as an electric toothbrush having an oscillating brush and having a function of flowing minute current to an oral cavity of a user for removing plaque, although the present invention is not limited to this particular device.

The device includes a tubular handle **10** and a brush head **60** extending from the handle **10** and carrying the brush **62** at its front end. The handle **10** is provided with an oscillating shaft **20** which driven by an electromagnetic actuator **20** mounted in the handle **10** together with a battery **12.** The actuator **22** is powered by the battery **12** and is configured to selectively oscillate the shaft **20** along its axis and about its axis at a rapid cycle of about 30,000 per minute. The selection is made by a selector **14** on the handle **10.** The shaft **20** extends from the upper end of the handle **10** for detachable engagement into a slot **61** at the rear end of the brush head **60** so that the brush **60** is driven by the shaft **20** to oscillate for cleaning the teeth.

A circuit board **30** is mounted within the handle **10** to give a boosting circuit which boots a voltage of the battery for driving the actuator **20** as well as a current limiting circuit which generates a minute current of 30 µA to 100 µA from the battery **12.**

The handle **10** is formed on its exterior surface with a touch electrode **50** for contact with a user's hand. The touch electrode **50** is made of a non-metal material filled with carbon fibers, and is cooperative with the handle **10** to define a water-tight interior space for accommodating therein the actuator **22,** the battery **12,** the circuit board **30,** and associated members. The touch electrode **50** is electrically connected to the current limiting circuit so as to be positively charged with regard to a brush electrode **64** disposed in the brush head **60** adjacent to the brush **62.** The brush electrode **64** is defined at a front end of a conductor **66** extending in the brush head **60,** and is exposed in an opening **68.** The conductor **66** has its rear end in electrical contact with the end of the shaft **20** and is connected to the current limiting circuit through the shaft **20** of electrically conductive material. Thus, the current limiting circuit flows the minute current through a path of the touch electrode **50,** the user's hand, gums, teeth, sputum, toothpaste, and the brush electrode **64** for removing the plaque.

The electrical connection of the touch electrode **50** to the current limiting circuit is made through a terminal **40,** a pin **42,** and a contact member **44**. The terminal **40** is in the form of a spring leaf supported to a block **41** fixed to the bottom of the handle **10** and is connected at its one end to the current limiting circuit. The other end of the terminal **40** is pressed against the pin **42** fitted in the wall of the handle **10.** The contact member **44** extends from the pin for pressed contact with the back of the touch electrode **50** for establishing the electrical connection of the touch electrode to the current limiting circuit. The shaft **20** is electrically connected to the current limiting circuit by use of a coil spring **24** which is fixed at its one end to the rear end of the shaft **20** and is connected at the other end to a terminal **46** leading to the current limiting circuit on the circuit board **30.**

The touch electrode **50** includes the carbon fibers which are dispersed in a non-electrically conductive resin such as ABC resin to give electrical conductivity for electrical connection with the terminal **40** in the handle **10.** Since the touch electrode **50** is devoid of any metal, it is free from elution of metallic ions while being kept in contact with the user's hand, and is therefore free from contamination of such metallic ions.

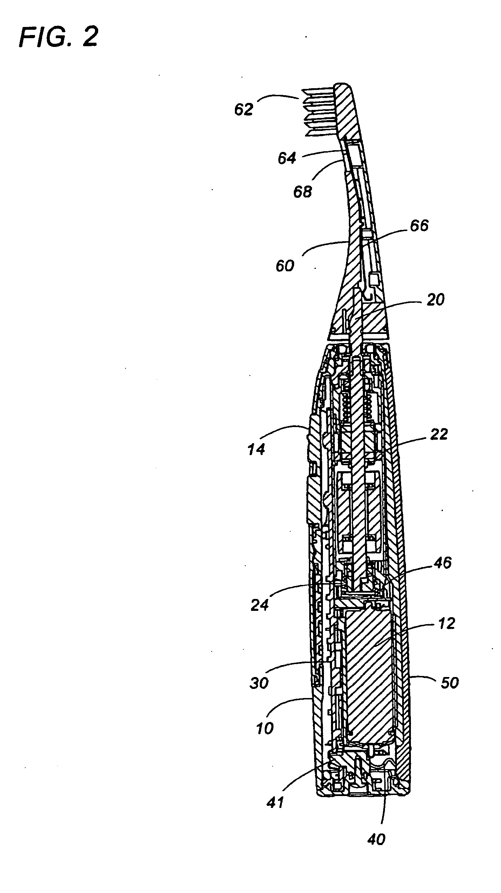

FIG. 2 shows an oral care device in accordance with an embodiment of the present invention which is identical to the above device except that the touch electrode **50** is molded together with the handle **10** to give a unitary molded structure. Like parts are designated by like reference numerals and duplicate explanation is deemed necessary. The touch electrode **50** is made of the non electrically conductive resin such as ABS resin dispersed with the carbon fibers, and is molded integrally with the handle **10** made of ABS resin such that a portion of the touch electrode **50** extends into the water-tight interior space for direct contact with the terminal **40** at the bottom of the handle **10.** The touch electrode **50** extends in an overlapping relation with a rear wall of the handle **10** except at a bottom end of the handle **10** so to be exposed to the interior space thereat.

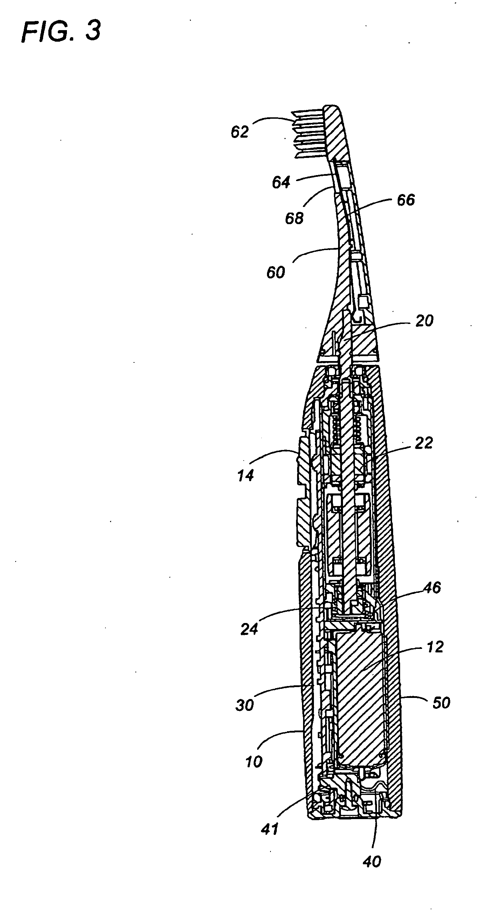

FIG. 3 shows a modification of the embodiment of FIG. 2 in which the touch electrode **50** is molded to constitute the wall of the handle **10** to be entirely exposed to the interior space for contact with the terminal **40.**

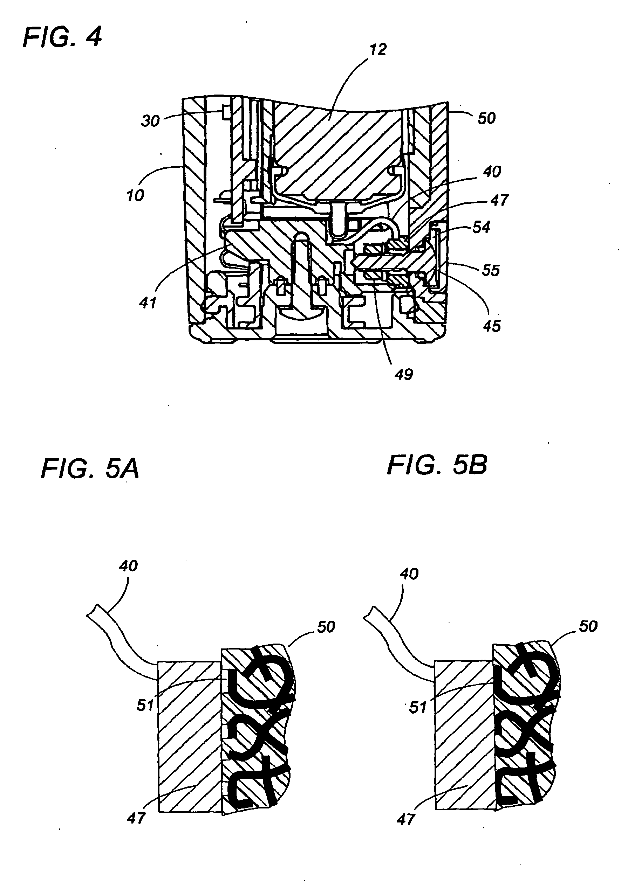

FIG. 4 shows another modification which is identical to the embodiment of FIG. 2 except that the touch electrode **50** is fastened to the terminal **40** by use of a screw **45.** The screw **45** extends through a bottom of a recess **54** formed in the outer surface of the touch electrode **50** into a nut **49** to hold a ring **47** between the nut and the touch electrode **50**. The ring **47** is made of an electrical conductive material connected to the terminal **40** and is held in pressed contact with the touch electrode **50** to make the electrical connection thereat. Therefore, even if the carbon fibers **51** happen to be receded from the rear surface of the touch electrode **50,** as shown in FIG. 5A, the tightening of the screw can squeeze the touch electrode 30, i.e., the non-electrical resin matrix to bring the carbon fibers **51** into intimate contact with the ring **47,** as shown in FIG. 5B, to make a reliable electrical connection. The recess **54** is fitted with a cap **55** of a non-electrical material for concealing the screw head within the recess **54.** The screw may be made of a non-electrical conductive material or of an electrically conductive material.

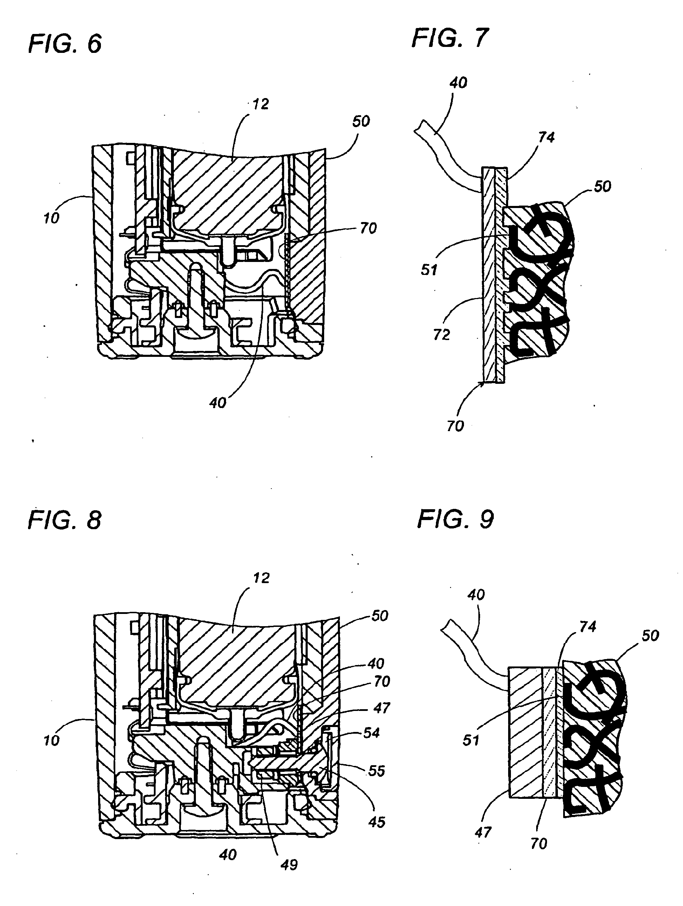

FIGS. 6 and 7 show a further modification which is identical to the embodiment of FIG. 2 except for the use of an electrically conductive adhesive in the form of a tape **70** connected to the terminal **40.** The tape **70** is composed of a film **72** of electrically conductive material leading from the terminal **40,** and a viscous adhesive coating **74** also of an electrically conductive material. As shown in FIG. 5, the viscous adhesive coating can permeate into possible cavities of the touch electrode **50** for successful contact with the carbon fibers **51** even if the carbon fibers happen to be hidden in the cavities as illustrated.

FIGS. 8 and 9 show a still further modification which is similar to the above modification of FIG. 4 but additionally use the tape **70** as utilized in the above modification of FIG. 6. Like parts are designated by like reference numerals and duplicate explanations are omitted. The adhesive tape **70** is held between the ring **47** and the touch electrode **50.** Thus, as shown in FIG. 9, the tightening of the screw **45** forces the viscous adhesive coating **74** to permeate into possible cavities of the touch electrode **50** for successful contact with the carbon fibers **51** even if the carbon fibers happen to be hidden in the cavities as illustrated.

Although the above embodiments and modifications are illustrated to have the positively charged touch electrode **50** with respect to the brush electrode **64,** the present invention is not limited to this specific arrangement and may be designed to charge the touch electrode **50** negative with respect to the brush electrode, in consideration of that the touch electrode **50** is likely to be wetted during the use and would certainly suffer from elution if the touch electrode **50** is made of a metallic material.

## Claims

1. An oral care device comprising:
a handle (10) provided with a touch electrode (50) for contact with a user's hand;
a brush head (60) extending from said handle and carrying a brush (62) configured to be placed into an oral cavity of a user, said brush head being provided with a brush electrode (64) being disposed adjacent to said brush and configured to come into electrical contact with portions or substances in the oral cavity; and
a power source (12) accommodated in said handle to apply a voltage difference between said touch electrode and said brush electrode;
said handle has a water-tight interior space within which said power source is disposed together with terminals (40, 46) respectively for electrical connection with said brush electrode and said touch electrode;
**characterized in that**
said touch electrode is made of non-metal material filled with carbon filaments,
and molded as an integral part of said handle,
said touch electrode is composed of a non-electrically conductive matrix within which said carbon filaments are dispersed,
said touch electrode being electrically connected to a corresponding one of said terminals by means of an electrically conductive adhesive (74) and/or a screw (45) extending from said touch electrode to said terminal.

2. An oral care device as set forth in claim 1, wherein said touch electrode is charged positive with respect to said brush electrode.

3. An oral care device as set forth in claim 1, wherein
said touch electrode has a portion which extends continuously into said interior space of said handle for electrical connection with the corresponding terminal.

4. An oral care device as set forth in claim 1, wherein
said electrically conductive adhesive is configured into a tape (70),
said touch electrode is fastened to said terminal by means of a screw (45).

5. An oral care device as set forth in claim 1, wherein
said screw has a screw head disposed in a recess (54) formed in a top surface of said touch electrode,
said recess is fitted with a cap (55) for concealing the screw head.

6. An oral care device as set forth in claim 1, wherein
said screw has a screw head disposed in a recess (54) formed in a top surface of said touch electrode, and
said recess is fitted with a cap (55) for concealing the screw head.

## Patentansprüche

1. Mundpflegevorrichtung, die Folgendes umfasst:
einen Griff (10), der mit einer Berührungselektrode (50) für Kontakt mit einer Benutzerhand versehen ist;
einen Bürstenkopf (60), der sich von dem Griff erstreckt und eine Bürste (62) trägt, die eingerichtet ist, in eine Mundkavität eines Benutzers platziert zu werden, wobei der Bürstenkopf mit einer Bürstenelektrode (64) versehen ist, die benachbart zu der Bürste angeordnet ist und eingerichtet ist, in elektrischen Kontakt mit Teilen oder Substanzen in der Mundkavität zu kommen; und
eine Stromquelle (12), die in dem Griff aufgenommen ist, um einen Spannungsunterschied zwischen der Berührungselektrode und der Bürstenelektrode anzulegen;
wobei der Griff einen wasserdichten Innenraum aufweist, innerhalb dessen die Stromquelle zusammen mit Anschlüssen (40, 46) für elektrische Verbindung mit der Bürstenelektrode beziehungsweise der Berührungselektrode angeordnet ist;
**dadurch gekennzeichnet, dass**
die Berührungselektrode aus einem Nichtmetallmaterial gemacht ist, das mit Kohlenstofffilamenten gefüllt ist,
und als ein integraler Bestandteil des Griffs ausgeformt ist,
wobei die Berührungselektrode aus einer nicht elektrisch leitenden Matrix gebildet ist, innerhalb deren sich die Kohlenstofffilamente verteilen,
wobei die Berührungselektrode mit einem entsprechenden der Anschlüsse mittels eines elektrisch leitenden Haftmittels (74) und/oder einer Schraube (45), die sich von der Berührungselektrode zu dem Anschluss erstreckt, elektrisch verbunden ist.

2. Mundpflegevorrichtung nach Anspruch 1, wobei die Berührungselektrode bezüglich der Bürstenelektrode positiv geladen ist.

3. Mundpflegevorrichtung nach Anspruch 1, wobei
die Berührungselektrode einen Abschnitt aufweist, der sich für elektrische Verbindung mit dem entsprechenden Anschluss in den Innenraum des Griffs kontinuierlich erstreckt.

4. Mundpflegevorrichtung nach Anspruch 1, wobei
das elektrisch leitende Haftmittel in einem Band (70) eingerichtet ist,
die Berührungselektrode an dem Anschluss mittels einer Schraube (45) befestigt ist.

5. Mundpflegevorrichtung nach Anspruch 1, wobei
die Schraube einen Schraubenkopf aufweist, der in einer Aussparung (54) angeordnet ist, die in einer oberen Oberfläche der Berührungselektrode geformt ist,
die Aussparung mit einer Kappe (55) zum Verdecken des Schraubenkopfes ausgerüstet ist.

6. Mundpflegevorrichtung nach Anspruch 1, wobei
die Schraube einen Schraubenkopf aufweist, der in einer Aussparung (54) angeordnet ist, die in einer oberen Oberfläche der Berührungselektrode geformt ist und
die Aussparung mit einer Kappe (55) zum Verdecken des Schraubenkopfes ausgerüstet ist.

## Revendications

1. Dispositif de soin oral comprenant:
une poignée (10) dotée d'une électrode tactile (50) pour être contact avec la main d'un utilisateur;
une tête (60) à brosse s'étendant de ladite poignée et portant une brosse (62) configurée de manière à être placée dans une cavité orale d'un utilisateur, ladite tête à brosse étant dotée d'une électrode (64) de brosse qui est disposée de manière adjacente à ladite brosse et configurée de manière à se mettre en contact électrique avec des parties ou des substances dans la cavité orale; et
une source (12) d'alimentation électrique logée dans ladite poignée pour appliquer une différence de tension entre ladite électrode tactile et ladite électrode de brosse;
ladite poignée possède un espace interne étanche à l'eau dans lequel ladite source d'alimentation électrique est disposée conjointement avec des bornes (40, 46) respectivement pour un raccordement électrique avec ladite électrode de brosse et ladite électrode tactile;
**caractérisé en ce que**
ladite électrode tactile est réalisée en un matériau non métallique rempli de filaments de carbone, et moulée venue de matière avec ladite poignée,
ladite électrode tactile se compose d'une matrice non-électriquement conductrice dans laquelle lesdits filaments de carbone sont dispersés,
ladite électrode tactile étant électriquement raccordée à une borne correspondante desdites bornes au moyen d'un adhésif (74) électriquement conducteur et/ou une vis (45) s'étendant de ladite électrode tactile à ladite borne.

2. Dispositif de soin oral selon la revendication 1, dans lequel ladite électrode tactile est positivement chargée par rapport à ladite électrode de brosse.

3. Dispositif de soin oral selon la revendication 1, dans lequel
ladite électrode tactile a une partie qui s'étend de manière continue dans ledit espace interne de ladite poignée pour un raccordement électrique avec la borne correspondante.

4. Dispositif de soin oral selon la revendication 1, dans lequel
ledit adhésif électriquement conducteur est configuré en bande (70),
ladite électrode tactile est fixée à ladite borne au moyen d'une vis (45).

5. Dispositif de soin oral selon la revendication 1, dans lequel
ladite vis a une tête de vis disposée dans une cavité (54) formée dans une surface supérieure de ladite électrode tactile,
ladite cavité est équipée d'un couvercle (55) pour cacher la tête de la vis.

6. Dispositif de soin oral selon la revendication 1, dans lequel
ladite vis a une tête de vis disposée dans une cavité (54) formée dans une surface supérieure de ladite électrode tactile, et
ladite cavité est équipée d'un couvercle (55) pour cacher la tête de la vis.
